(19) Europäisches Patentamt
European Patent Office
Office européen des brevets

(11) Veröffentlichungsnummer: **0 044 394**
**A2**

(12) # EUROPÄISCHE PATENTANMELDUNG

(21) Anmeldenummer: 81104210.0

(22) Anmeldetag: 02.06.81

(51) Int. Cl.³: C 07 D 249/08
C 07 D 233/56, C 07 D 233/92
A 01 N 43/64, A 01 N 43/50

(30) Priorität: 11.06.80 DE 3021834

(43) Veröffentlichungstag der Anmeldung: 27.01.82 Patentblatt 82/4

(84) Benannte Vertragsstaaten: AT BE CH DE FR GB IT LI LU NL SE

(71) Anmelder: BASF Aktiengesellschaft
Carl-Bosch-Strasse 38
D-6700 Ludwigshafen(DE)

(72) Erfinder: Hagen, Helmut, Dr.
Max-Slevogt-Strasse 17E
D-6710 Frankenthal(DE)

(72) Erfinder: Ziegler, Hans, Dr.
Am Speyerweg 48
D-6704444 Mutterstadt(DE)

(72) Erfinder: Mappes, Celia Joan, Dr.
Wiesenweg 145
D-6721 Westheim(DE)

(72) Erfinder: Pommer, Ernst-Heinrich, Dr.
Berliner Platz 7
D-6703 Limburgerhof(DE)

(54) **Azolyl-4-nitro-2-trichlormethylbenzolsulfone, ihre Herstellung und Verwendung.**

(57) Die Erfindung betrifft Azolyl-4-nitro-2-trichlormethylphenylsulfone der Formel

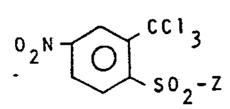

worin Z ein Triazol-3-yl-Rest oder die Gruppe

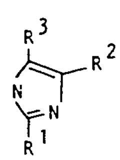

ist, in der

$R^1$ ein Wasserstoffatom, einen gegebenenfalls substituierten araliphatischen Rest mit 7 bis 9 Kohlenstoffatomen oder einen gegebenenfalls substituierten aromatischen Rest,

$R^2$ ein Wasserstoffatom, einen aliphatischen Rest oder -falls $R^3$ ein Wasserstoffatom ist - eine Nitrogruppe und

$R^3$ ein Wasserstoffatom, einen aliphatischen Rest oder -falls $R^2$ ein Wasserstoffatom ist - eine Nitrogruppe bedeuten, ihre Herstellung, ihre Verwendung als Fungizide und Nitrifaktionshemmer und Mittel dafür.

EP 0 044 394 A2

Croydon Printing Company Ltd.

Azolyl-4-nitro-2-trichlormethylbenzolsulfone, ihre Herstellung und Verwendung

Die vorliegende Erfindung betrifft neue Azolyl-4-nitro-2-trichlormethylbenzolsulfone, Verfahren zu ihrer Herstellung, fungizide Mittel, die diese Verbindungen enthalten, Verfahren zur Bekämpfung von Fungi mit diesen Verbindungen sowie die Herstellung fungizider Mittel aus den neuen Substanzen.

Es ist bekannt, N-Trichlormethylthiophthalimid (Chemical Week 1972 June 21, Seite 63) Tetramethylthiuramdisulfide (Chemical Week 1972, July 26, Seite 39) oder 2-Thiocyanomethylthiobenzothiazol (Farm chemicals Handbook 1976, Seite D 43) als Fungizide zu verwenden. Ihre Wirkung ist jedoch nicht gegen alle pathogene Pilzstämme befriedigend und gegen viele unbefriedigend.

Es wurde nun gefunden, daß Azolyl-4-nitro-2-trichlormethylbenzolsulfone der Formel I

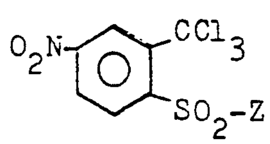 (I),

worin Z ein Triazol-3-yl-Rest oder die Gruppe

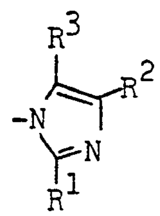

ist, in der

WK/BL

$R^1$ ein Wasserstoffatom, einen gegebenenfalls substituierten aliphatischen Rest mit 1 bis 16 Kohlenstoffatomen, einen gegebenenfalls substituierten araliphatischen Rest mit 7 bis 9 Kohlenstoffatomen oder einen gegebenenfalls substituierten aromatischen Rest,

$R^2$ ein Wasserstoffatom, einen aliphatischen Rest oder - falls $R^3$ ein Wasserstoffatom ist - eine Nitrogruppe und

$R^3$ ein Wasserstoffatom, einen aliphatischen Rest oder - falls $R^2$ ein Wasserstoffatom ist - eine Nitrogruppe

bedeuten, eine bessere fungizide Wirkung haben.

Als Substituenten am Rest $R^1$ kommen vor allem Hydroxy-, Nitro- und Halogen (insbesondere Chlor-)-reste in Frage. $R^1$ bedeutet vorzugsweise ein Wasserstoffatom, eine Alkylgruppe mit 1-4 Kohlenstoffatomen oder den Phenylrest.

Als aliphatische Reste für $R^2$ und $R^3$ kommen insbesondere Alkylreste mit 1-4 Kohlenstoffatomen in Betracht.

Als Amidreste kommen daher besonders die Reste von Imidazol, 2-Isopropylimidazol, 2-Ethylimidazol, Triazol, 4(5)-Nitroimidazol, 2-Phenylimidazol, 2-Methyl-4(5)-nitroimidazol, 2-Ethyl-4(5)-nitroimidazol, 2-Isopropyl-4(5)-nitroimidazol, 2-Benzylimidazol und 2-Phenyl-4(5)-nitroimidazol in Betracht.

Die Herstellung der erfindungsgemäßen Verbindungen erfolgt durch Umsetzung von 4-Nitro-2-trichlormethylbenzolsulfonsäurechlorid der Formel III mit Triazol oder einem Imidazol der Formel II, in der $R^1$, $R^2$ und $R^3$ die

für Formel I oben angegebenen Bedeutungen haben in einem inerten Lösungsmittel und gegebenenfalls in Gegenwart einer säurebindenden Verbindung bei Temperaturen von -40° bis 120°C.

Die Umsetzung läßt sich durch folgende Reaktionsgleichung beschreiben.

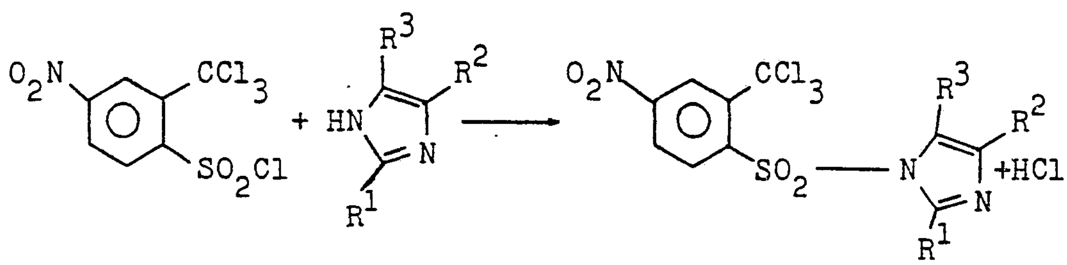

(III) (II) (I)

Die Umsetzung wird vorzugsweise in inerten Lösungsmitteln durchgeführt. Solche sind chlorierte Kohlenwasserstoffe - wie Dichlormethan -, Carbonsäuren - wie Essigsäure -, gesättigte oder cyclische Ether, aliphatische Carbonsäureester, aliphatische und aromatische Kohlenwasserstoffe.

Von den genannten Lösungsmitteln sind Dichlormethan, Essigsäureethylester und Toluol bevorzugt.

Es empfiehlt sich, die Umsetzung in Gegenwart säurebindender Verbindungen vorzunehmen, z.B. tertiäre Amine - wie Triethylamin -, Pyridin, anorganische Basen - wie NaOH, $Na_2CO_3$ -.

Der für die Umsetzung bevorzugte Temperaturbereich liegt bei 0 bis 60°C.

Die Ausgangsverbindung 4-Nitro-2-trichlormethylbenzolsulfonsäurechlorid (III) läßt sich durch Oxidation von

4-Nitro-2-trichlormethylbenzolsulfensäurechlorid (DE-OS 27 21 917) mit Chlor in Essigsäure und in Gegenwart einer äquivalenten Menge Wasser bei 20 bis 120°C herstellen:

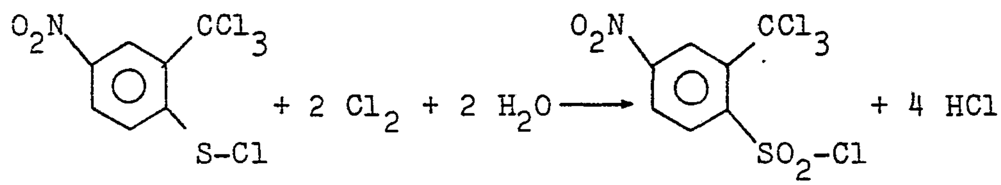

(III)

Überraschenderweise treten bei dieser Reaktionsführung keine Veränderungen der übrigen funktionellen Gruppen auf.

Die folgenden Beispiele erläutern die Herstellung der neuen Substanzen:

A. Herstellung von III

In eine Lösung von 614 Teilen 4-Nitro-2-trichlormethylbenzolsulfensäurechlorid und 72 Teilen Wasser in 2000 Teilen Eisessig werden bei 80°C bis zur Entfärbung mindestens 142 Teile Chlor eingeleitet.

Man läßt 1 Stunde bei 80°C und 12 Stunden bei Raumtemperatur nachreagieren. Das Lösungsmittel wird im Vakuum entfernt, der Rückstand in Dichlormethan aufgenommen und mit Wasser gewaschen. Nach Trocknen und Einengen der Dichlormethanphase erhält man 602 g (89%) 4-Nitro-2-trichlormethylbenzolsulfonsäurechlorid, Fp = 68°C.

B. Herstellung der Endprodukte

Beispiel 1

87,9 g %-Nitro-2-trichlormethylphenylsulfonsäurechlorid gelöst in 200 ml Methylenchlorid werden bei 10° (mit 27 g Imidazol in 400 ml Methylenchlorid umgesetzt. Anschließend wird 12 Stunden bei Raumtemperatur nachgerührt. Die organische Phase wird dreimal mit Wasser gewaschen, getrocknet und eingeengt. Nach Umkristallisation aus Ether erhält man 55 g Imidazol-1-yl-(4-nitro-2-trichlormethylphenyl)-sulfon, Fp = 125°C.

| | C | H | N | S | Cl |
|---|---|---|---|---|---|
| ber. | 32,4 | 1,6 | 11,3 | 8,7 | 27,7 |
| gef. | 32,5 | 1,8 | 11,4 | 8,5 | 28,6 |

Beispiel 2

67,8 g 4-Nitro-2-trichlormethylphenylsulfonsäurechlorid werden analog Beispiel 1 mit 32,8 g 2-Methylimidazol umgesetzt. Man erhält nach Umkristallisation aus Ether 65 g 2-Methylimidazol-1-yl-(4-nitro-2-trichlormethylphenyl)sulfon, Fp = 168 - 169°C.

| | C | H | | S | Cl |
|---|---|---|---|---|---|
| ber. | 34,4 | 2,1 | 10,9 | 8,3 | 27,7 |
| gef. | 34,4 | 2,2 | 11,0 | 8,4 | 27,4 |

Beispiel 3

67,8 g 4-Nitro-2-trichlormethylbenzolsulfonsäurechlorid werden analog Beispiel 1 mit 44 g 2-Isopropylimidazol umgesetzt. Man erhält aus Ether 63 g (76%) 2-Isopropyl-1--yl-(4-nitro-2-trichlormethylphenyl)sulfon, Fp = 120°C.

| | C | H | N | S | Cl |
|---|---|---|---|---|---|
| ber. | 37,8 | 2,9 | 10,2 | 7,8 | 25,8 |
| gef. | 38,1 | 3,0 | 10,4 | 7,6 | 25,6 |

Beispiel 4

67,8 g 4-Nitro-2-trichlormethylbenzolsulfonsäurechlorid werden analog Beispiel 1 mit 38,4 g 2-Ethylimidazol umgesetzt. Aus Methanol erhält man 63 g (79%) 2-Ethylimidazol-1-yl-(4-nitro-2-trichlormethylphenyl)sulfon, Fp = 139°C.

| | C | H | N | S | Cl |
|---|---|---|---|---|---|
| ber. | 36,1 | 2,5 | 10,5 | 8,0 | 26,7 |
| gef. | 36,1 | 2,5 | 10,5 | 7,9 | 16,8 |

Beispiel 5

67,8 g 4-Nitro-2-trichlormethylbenzolsulfonsäurechlorid werden analog Beispiel 1 mit 57,6 g 2-Phenylimidazol umgesetzt. Man erhält aus Methanol 67 g (75 %) 2-Phenylimidazol-1-yl-(4-nitro-2-trichlormethylphenyl)sulfon, Fp = 179°C.

| | C | H | N | S | Cl |
|---|---|---|---|---|---|
| ber. | 43,0 | 2,2 | 9,4 | 7,2 | 23,9 |
| gef. | 43,2 | 2,5 | 9,4 | 7,1 | 23,7 |

Beispiel 6

33,9 g 4-Nitro-2-trichlormethylbenzolsulfonsäurechlorid gelöst in 200 ml Dichlormethan werden bei 10°C mit einer Lösung von 5,5 g 1,3,4-Triazol und 14 ml Triethylamin in 400 ml Dichlormethan umgesetzt. Die Dichlormethanphase wird nach 12-stündigem Rühren bei Raumtemperatur dreimal

mit Wasser gewaschen, getrocknet und einrotiert. Man erhält aus Methanol 31 g (83%) Triazol-3-yl-(4-nitro-2-trichlormethylphenyl)sulfon, Fp = 150 °C.

| | C | H | N | S | Cl |
|---|---|---|---|---|---|
| ber. | 29,1 | 1,3 | 15,1 | 8,6 | 28,7 |
| gef. | 29,3 | 1,5 | 14,8 | 8,5 | 28,7 |

Beispiel 7

33,9 g 4-Nitro-2-trichlormethylbenzolsulfonsäurechlorid werden analog Beispiel 6 mit 11,3 g 4(5)-Nitroimidazol umgesetzt. Man erhält aus Methanol 26 g (62)% 4(5)-Nitroimidazol-1-yl-(4-nitro-2-trichlormethylphenyl)sulfon, Fp = 186°C.

| | C | H | N | S | Cl |
|---|---|---|---|---|---|
| ber. | 28,9 | 1,2 | 13,5 | 7,7 | 25,6 |
| gef. | 30,0 | 1,5 | 13,6 | 7,5 | 25,4 |

Beispiel 8

33,9 g 4-Nitro-2-trichlormethylbenzolsulfonsäurechlorid werden analog Beispiel 6 mit 12,7 g 2-Methyl-4(5)-nitroimidazol umgesetzt. Aus Methanol erhält man 28 g 2-Methyl-4(5)-nitroimidazol-1-yl-(4-nitro-2-trichlormethylphenyl)-sulfon, Fp = 221°C.

| | C | H | N | S | Cl |
|---|---|---|---|---|---|
| ber. | 28,9 | 1,2 | 13,5 | 7,7 | 25,6 |
| gef. | 28,9 | 1,3 | 13,5 | 7,6 | 25,7 |

Beispiel 9

33,9 g 4-Nitro-2-trichlormethylbenzolsulfonsäurechlorid werden analog Beispiel 6 mit 14,5 g 2-Ethyl-4(5)-nitroimidazol umgesetzt. Aus Methanol erhält man 33 g (74 %) 2-Ethyl-4(5)-nitroimidazol-1-yl-(4-nitro-2-trichlormethylphenyl)sulfon, Fp = 194°C.

| | C | H | N | S | Cl |
|---|---|---|---|---|---|
| ber. | 32,5 | 2,0 | 12,6 | 7,2 | 24,0 |
| gef. | 32,4 | 2,3 | 12,4 | 7,2 | 24,2 |

Beispiel 10

33,9 g 4-Nitro-2-trichlormethylbenzolsulfonsäurechlorid werden analog Beispiel 6 mit 15,5 g 2-Isopropyl-4(5)-nitroimidazol umgesetzt. Man erhält aus Ether 37 g (81 %) 2-Isopropyl-4(5)-nitroimidazol-1-yl-(4-nitro-2-trichlormethylphenyl)sulfon, Fp = 156°C.

| | C | H | N | S | Cl |
|---|---|---|---|---|---|
| ber. | 34,1 | 2,4 | 12,2 | 7,0 | 23,3 |
| gef. | 34,3 | 2,6 | 12,2 | 6,9 | 23,5 |

Die neuen Verbindungen besitzen eine sehr gute fungitoxische Wirksamkeit gegenüber verschiedenen Arten von Schadpilzen, die im Pflanzenschutz oder Materialschutz wirtschaftlich bedeutend sind. Das betrifft insbesondere Septoria nodorum und Septoria tritici an Weizen, Helminthosporium-Arten an Getreide, Pythium-Arten an Leguminosen und Baumwolle, Cercospora-Arten an Erdnüssen, Rüben, Kaffee, Mycosphaerella an Bananen, Piricularia an Reis. Darüber hinaus zeichnen sich einige Verbindungen durch eine sehr gute bakterizide Wirksamkeit, beispielsweise gegen Staphylococcus aureus, Nitrobacter und Nitrosomonas aus. Sie können daher auch als Nitrifikationshemmer eingesetzt werden.
Die fungiziden Mittel enthalten 0,1 bis 95 % (Gew.-%) Wirkstoff, vorzugsweise 0,5 bis 90 %. Die Aufwandmengen liegen je nach Art des gewünschten Effektes zwischen 0,001 und 3 kg oder mehr, vorzugsweise jedoch zwischen 0,01 und 1 kg Wirkstoff/ha. Bei der Anwendung der Wirkstoffe im Materialschutz z.B. als Fungizide für Anstrichfarben, betragen die Aufwandmengen 0,5 bis 5 % Wirkstoff, bezogen auf das Gesamtgewicht der zu konservierenden Stoffe, beispielsweise Farben. Die neuen Wirkstoffe können auch als fungizid wirksame Bestandteile öliger Holzschutzmittel zum Schutz von Holz gegen holzzerstörende oder holzverfärbende Pilze eingesetzt werden. Die Anwendung erfolgte in der Weise, daß man das Holz mit diesen Mitteln behandelt, beispielsweise tränkt oder anstreicht.

Die Anwendung erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Bei-

spiel Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Dispergier- oder Emulgiermittel und evtl. Lösungsmittel oder Öl bestehende Konzentrate hergestellt werden, die zur Verdünnung mit Wasser geeignet sind.

An oberflächenaktiven Stoffen sind zu nennen: Alkali-, Erdalkali-, Ammoniumsalze von Ligninsulfonsäure, Naphthalinsulfonsäuren, Phenolsulfonsäure, Alkylarylsulfonate, Alkylsulfonate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, $\alpha$-Octylphenol, $\alpha$-Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes

Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Silikate, Talkum, Kaolin, Kalkstein, Kalk, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehle, Cellulosepulver und andere feste Trägerstoffe.

Die Wirkstoffe können auch mit anderen bekannten Fungiziden gemischt werden. In vielen Fällen erhält man dabei eine Vergrößerung des fungziden Wirkungsspektrums; bei einer Anzahl dieser Fungzidmischungen treten synergistische Effekte auf, d.h. die fungizide Wirksamkeit des Kombinationsproduktes ist größer als die der addierten Wirksamkeit der Einzelkomponenten.
Fungizide, die mit den erfindungsgemäßen 4-Nitro-2-trichlormethylphenylsulfenamiden kombiniert werden können, sind beispielsweise:

Ferridimethyldithiocarbamat,
Zinkdimethyldithiocarbamat,
Manganethylenbisdithiocarbamat,
Mangan-zink-ethylendiamin-bis-dithiocarbamat,
Zinkethylenbisdithiocarbamat,

Tetramethylthiuramdisulfid,
3,5-Dimethyl-1,3,5-2H-tetrahydrothiadiazin-2-thion,
Ammoniak-Komplex von Zink-(N,N'-ethylen)-bis-dithiocarbamat und
N,N'-Polyethylen-bis-(thiocarbamoyl)-disulfid,
Zink-(N,N'-propylen)-bis-dithiocarbamat,
Ammoniak-Komplex von Zink-(N,N'-propylen-bis-dithiocarbamat) und
N,N'-Polypropylen-bis-(thiocarbamoyl)-disulfid,
Dinitro-(1-methylheptyl)-phenyl-crotonat,
2-sec.-Butyl-4,6-dinitrophenyl-3,3-dimethylacrylat,
2-sec.-Butyl-4,6-Dinitrophenylisopropylcarbonat,
2,4,5-Trichlorphenol
Pentachlorphenol,
Barium-Salz von Pentachlorphenol,
Pentachlorphenyl-acetat,
Pentachlorbenzylalkohol,
Di-(5-chloro-2-hydroxyphenyl)-methan,
Phenyl-(5-chloro-2-hydroxyphenyl)-methan,
N-Trichlormethylthio-tetrahydrophthalamid,
N-Trichlormethylthio-phthalimid,
N-Fluordichlormethylthio-phthalimid,
N-(1,1,2,2-Tetrachlorethylthio)-tetrahydrophthalimid,
2-Heptadecyl-2-imidazolinacetat,
2,4-Dichlor-6-(o-chloranilino)-s-triazin,
Diethylphthalimidophosphorothioat,
5-Amino-1-[bis-(dimethylamino)-phosphinyl]-3-phenyl-1,2,4-triazol,
5-Ethoxy-3-trichlormethyl-1,2,4-thiadiazol,
2,3-Dicyano-1,4-dithioanthrachinon,
Chinoxalin-2,3-cycl.-trithiocarbonat,
1-(Butylcarbamoyl)-2-benzimidazol-carbaminsäuremethylester,
2-Methoxycarbonylamino-benzimidazol,
2-Rhodanmethylthio-benzthiazol,

4-(2-Chlorphenylhydrazono)-3-methyl-5-isoxazolon,
1-(1,2,4-Triazolyl-1')-[1-(4'-chlorphenoxy)]-3,3-dimethyl-butan-2-on,
1-(1-Imidazolyl)-2-allyloxy-2-(2,4-dichlorphenyl)-ethan,
2-(O,O-Diethyl-thionophosphoryl)-5-methyl-6-carbethoxy-pyrazol-(1,5a)-pyrimidin,
Pyridin-2-thiol-1-oxid,
8-Hydroxychinolin bzw. dessen Kupfer-Salz,
5,5-Dimethyl-3-(3,5-dichlorphenyl)-2,4-dioxo-1,3-oxazolidin,
5-Methyl-5-vinyl-3-(3,5-dichlorphenyl)-2,4-dioxo-1,3-oxazolidin,
2-[Furyl-(2)]-benzimidazol,
Piperazin-1,4-diyl-bis-1-(2,2,2-Trichlor-ethyl)-formamid,
2-[Thiazolyl-(4)]-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-1-thiorueido)-benzol,
Dodecylguanidinacetat,
3-[2-(3,5-Dimethyl-2-oxycyclohexyl)-2-hydroxyethyl]-glutarimid,
Hexachlorbenzol,
N-Dichlorfluoromethylthio-N',N'-dimethyl-N-phenyl-schwefelsäurediamid,
N-Dichlorfluormethylthio-N-methyl-N'-methyl-N'-phenyl-schwefelsäurediamid,
2,4,5,6-Tetrachlor-isophthalonitril,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
2,3-Dichlor-1,4-naphthochinon,
1,4-Dichlor-2,5-dimethoxybenzol,
p-Dimethylaminobenzol-diazonatriumsulfonat,
2-Chlor-1-nitro-propan,

Polychlornitrobenzole wie Pentachlornitrobenzol,
Methylisothiocyanat,
Triphenyl-zinn-acetat,
fungizide Antibiotika wie Griseofulvin oder Kasugamcycin,
Tetrafluordichloraceton,
1-Phenylthiosemicarbazid,
Aluminiumkomplex des N'-Hydroxy-N-cyclohexyl-diazeniumoxids,
Bordeauxmischung,
nickelhaltige Verbindungen und Schwefel,
Mercaptobenzthiazol,
Methyl-N-(2,6-dimethyl)-N-(2-furoyl)-alaninat,
Methyl-N-(2,6-dimethyl)-N-(2-methoxyacetyl)-alaninat,
2-Cyano-N-[(ethylamino)carbonyl]-2-(methoximino)-acetamid,
ß-(4-Chlorphenoxy)-α-(1,1-dimethyl-1H-1,2,4-triazol-1-ethanol,
Benzisothiazolon.

Die Zumischung dieser Mittel zu den erfindungsgemäßen Fungiziden kann im Gewichtsverhältnis 1:10 bis 10:1 erfolgen. Sie können gegebenenfalls auch erst unmittelbar vor der Anwendung (Tankmischung) zugesetzt werden.

Die folgenden biologischen Beispiele zeigen die Wirkung der neuen Substanzen.

Beispiel A

Wirksamkeit gegen Septoria nodorum an Weizen

Blätter von in Töpfen gewachsenen Weizenpflanzen der Sorte "Jubilar" werden mit wäßrigen Spritzbrühen, die 0,1 % (Gewichtsprozent) Wirkstoff emulgiert enthalten, tropfnaß

gespritzt. Nach dem Antrocknen des Spritzbelages werden die Pflanzen mit einer Sporenaufschwemmung des Pilzes Septoria nodorum besprüht und bei 16 bis 18°C in eine Kammer mit hoher Luftfeuchtigkeit gestellt, um optimale Bedingungen für die Pilzentwicklung zu erhalten. Nach 14 Tagen hat sich die Krankheit auf den unbehandelten Kontrollpflanzen so stark entwickelt, daß die entstandenen Blattflecken den überwiegenden Teil der Blätter bedecken.

In diesem Versuch zeigten insbesondere die Substanzen der Beispiele 1, 2, 3, 4 und 10 eine bessere Wirkung als die Vergleichsubstanz Chlorothalonil (= 2, 4, 5, 6 -Tetrachlorisophthalodinitril).

Beispiel B

Wirksamkeit gegen Phytophthora infestans an Tomaten

Blätter von Topfpflanzen der Sorte "Große Fleischtomate" werden mit wäßrigen Suspensionen, die 0,025 % (Gewichtsprozent) Wirkstoff enthalten, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

In diesem Versuch zeigten insbesondere die Substanzen der Beispiele 1, 4, 5 und 6 eine bessere Wirkung als die Vergleichssubstanz Captan (= N-Trichlormethylthiotetrahydrophthalimid).

## Patentansprüche

1. Azolyl-4-nitro-2-trichlormethylphenylsulfone der Formel I

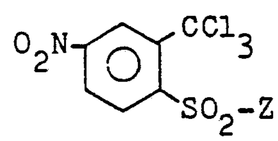

(I),

worin Z ein Triazol-3-yl-Rest oder die Gruppe

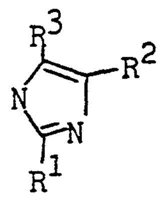

ist, in der

$R^1$ ein Wasserstoffatom, einen gegebenenfalls substituierten aliphatischen Rest mit 1 bis 16 Kohlenstoffatomen einen gegebenenfalls substituierten araliphatischen Rest mit 7 bis 9 Kohlenstoffatomen oder einen gegebenenfalls substituierten aromatischen Rest,

$R^2$ ein Wasserstoffatom, einen aliphatischen Rest oder - falls $R^3$ ein Wasserstoffatom ist - eine Nitrogruppe und

$R^3$ ein Wasserstoffatom, einen aliphatischen Rest oder - falls $R^2$ ein Wasserstoffatom ist - eine Nitrogruppe bedeuten.

2. Verfahren zur Herstellung von Verbindungen der Formel I, <u>dadurch gekennzeichnet,</u> daß man 4-Nitro-2-trichlormethylbenzolsulfonsäurechlorid mit Triazol oder einem Imidazol der Formel II

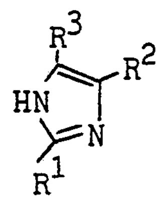

(II),

in der $R^1$, $R^2$ und $R^3$ die für die Formel I angegebenen Bedeutungen haben, umsetzt.

3. Fungizides Mittel, enthaltend mindestens eine Verbindung der Formel I gemäß Anspruch 1.

4. Fungizides Mittel, enthaltend mindestens eine Verbindung der Formel I gemäß Anspruch 1 und einen festen oder flüssigen Trägerstoff.

5. Verfahren zur Bekämpfung von Fungi, dadurch gekennzeichnet, daß man mindestens eine Verbindung der Formel I gemäß Anspruch 1 auf diese einwirken läßt.

6. Verfahren zur vorbeugenden Bekämpfung von Fungi dadurch gekennzeichnet, daß man mindestens eine Verbindung der Formel I gemäß Anspruch 1 auf durch Fungizid-Befall bedrohte Flächen, Pflanzen oder Saatgüter einwirken läßt.

7. Verfahren zur Herstellung von fungiziden Mitteln, dadurch gekennzeichnet, daß mindestens eine Verbindung der Formel I gemäß Anspruch 1 mit festen oder flüssigen Trägerstoffen mischt.

8. Nitrifikationshemmer, enthaltend mindestens eine Verbindung der Formel I gemäß Anspruch 1.

9. Nitrifikationshemmer, enthaltend mindestens eine Verbindung der Formel I gemäß Anspruch 1 und einen festen oder flüssigen Trägerstoff.